# EUROPÄISCHE PATENTANMELDUNG

(11) **EP 0 778 027 A2**
(43) Veröffentlichungstag der Anmeldung: **11.06.1997**
(21) Anmeldenummer: 96119255.6
(22) Anmeldetag: 01.12.1996
(51) Int. Cl.: A61K 33/00

(54) **Verwendung von Kieselerde zur Herstellung von Arzneimitteln**

(30) Priorität: 04.12.1995 AT 1972/95
(71) Anmelder: Wachter, Helmut, Univ.-Prof. Dr., A-6020 Innsbruck (AT)
(72) Erfinder: Wachter, Helmut, Univ.-Prof. Dr., A-6020 Innsbruck (AT)
(74) Vertreter: Torggler, Paul, Dr.

(57) **Zusammenfassung**

Verwendung von Kieselerde zur Herstellung eines Arzneimittels für Menschen zur Beeinflussung des Lipidstoffwechsels, insbesondere zur Senkung der Cholesterinkonzentration im menschlichen Blut.

## Beschreibung

Es ist bekannt, Kieselerde oral zu verabreichen, um eine gesunde straffe Haut, feste Nägel und glänzende Haare zu erhalten. Es ist ferner bekannt, daß durch die Einnahme von Kieselerde Knochen von innen gefestigt werden.

Demgegenüber besteht die Erfindung in der Verwendung von Kieselerde - ausgenommen als Adsorbens für organische therapeutische Wirkstoffe - zur Herstellung eines Arzneimittels für Menschen zur Beeinflussung des Lipidstoffwechsels, insbesondere zur Senkung der Cholesterinkonzentration im menschlichen Blut.

Unter Kieselerde versteht man nach Römpp, Chemie Lexikon, 9. Aufl., Band 3 (1990) im wesentlichen aus SiO₂ bestehende Mineralien, insbesondere solche, die aus in Schichten abgelagerten Panzern von Radiolarien und Diatomeen bestehen (Diatomeenerde).

Es wurde bereits vorgeschlagen, SiO₂ als Adsorbens für einen organischen Wirkstoff zur Erniedrigung des Cholesterinspiegels im Blut zu verwenden (US-A 4,185,088, DE-A 28 06 707). Auch die Verwendung von Organosiliciumverbindungen zur Beeinflussung des Glycometabolismus und Lipidmetabolismus wurde bereits vorgeschlagen (JP-A 57-128693). Bekannt ist ferner die Verwendung von Aluminiumsilicat als Bestandteil in einem Calciumhexosephosphat-Präparat zur Beeinflussung des Lipidstoffwechsels (JP-A 63-198630.

Der Erfindung liegt die Erkenntnis zugrunde, daß sich Kieselerde, insbesondere Diatomeenerde, nicht nur als Adsorbens für cholesterinsenkende andere Wirkstoffe (insbesondere organische Wirkstoffe) eignet, sondern dann, wenn es nicht als Adsorbens eingesetzt wird, selbst zur Senkung des Cholesteringehaltes im menschlichen Blut geeignet ist. Die erfindungsgemäße Verwendung von im wesentlichen aus amorphem SiO₂ bestehender Kieselerde unterscheidet sich auch von der bekannten Verwendung von Organosiliciumverbindungen oder kristallinen Silicaten, wobei besonders auf die Nebenwirkungsfreiheit sowie die gute Resorbierbarkeit von Kieselerde hinzuweisen ist.

Während bei der bekannten therapeutischen Verwendung von Kieselerde für Haut, Nägel oder Zähne das Silicium in die Struktur der Haut, der Nägel oder der Zähne eingebaut wird, ist für die erfindungsgemäße Indikation ein völlig anderer Wirkmechanismus verantwortlich. Einerseits werden offenbar mit der Nahrung aufgenommene Lipide im Magen-Darm-Trakt an der oberflächenaktiven Kieselerde gebunden und über den Stuhl abgeführt, anderseits wird durch die Kieselerde im Darm Gallensäure gebunden und mit dem Stuhl ausgeschieden, wodurch die intestinale Rückresorption vermindert und als Kompensationsmechanismus von der Leber mehr Cholesterin in Gallensäure übergeführt wird. Die Kieselerde kann also im Organismus (Magen-Darm-Trakt) durchaus für die dort vorhandenen, vom Körper produzierten Substanzen, z.B. für die dort vorhandene Gallensäure, als Adsorbens dienen, was nicht im Widerspruch zur Lehre der Erfindung steht, daß außerhalb des Organismus, d.h. bei der Zubereitung des Arzneimittels vor dessen Verabreichung die Kieselerde nicht bloß als Adsorbens für andere (organische) Wirkstoffe eingesetzt wird (Disclaimer).

Die interne Erprobung bei Patienten mit erhöhtem Cholesteringehalt im Blut hat ergeben, daß nach Verabreichung von Kieselerde innerhalb weniger Wochen eine signifikante Senkung des Cholesteringehaltes erzielt werden kann.

Die Kieselerde, eine pulverförmige Substanz im wesentlichen aus SiO₂ bestehend, kann z.B. in Kapseln, die unterschiedlich im Magen- oder Darm-Trakt aufgelöst werden, oder aber gegebenenfalls in Kombination mit Geschmacks-Ingredienzien als Pulver bzw. suspendiert in Flüssigkeiten verabreicht werden.

Die Verabreichung des Medikaments soll kurmäßig über einen Zeitraum von einigen Wochen bis einigen Monaten erfolgen, vorzugsweise unter Verlaufskontrollen.

Die tägliche Dosis kann beispielsweise 3 x 200 bis 300 mg SiO₂, vorzugsweise 2 x etwa 250 mg SiO₂ betragen. Die Einnahme soll vorzugsweise zu den Mahlzeiten erfolgen.

## Patentansprüche

1. Verwendung von Kieselerde, insbesondere Diatomeenerde - ausgenommen als Adsorbens für organische therapeutische Wirkstoffe - zur Herstellung eines Arzneimittels für Menschen zur Beeinflussung des Lipidstoffwechsels, insbesondere zur Senkung der Cholesterinkonzentration im menschlichen Blut.
